# EUROPEAN PATENT SPECIFICATION

(11) **EP 0 825 839 B1**
(45) Date of publication and mention of the grant of the patent: **30.07.2003**
(21) Application number: 96914687.7
(22) Date of filing: 17.05.1996
(51) Int. Cl.: A61F 2/06

(54) **STENT DEPLOYMENT CATHETER WITH COLLAPSIBLE SHEATH**
KATHETER MIT FALTBARER HÜLLE ZUM AUSBRINGEN EINES STENTS
CATHETER POUR PLACER UNE PROTHESE INTRAVASCULAIRE AVEC UNE GAINE REPLIABLE

(30) Priority: 18.05.1995 US 444822
(43) Date of publication of application: 04.03.1998
(73) Proprietor: Scimed Life Systems, Inc., Maple Grove, Minnesota 55311-1566 (US)
(72) Inventor: ST. GERMAIN, Jon, P., Elk River, MN 55330 (US); OLSON, Scott, A., Zimmerman, MN 55398 (US)
(74) Representative: Graalfs, Edo, Dipl.-Ing.
(86) International application number: PCT/US96/07143
(87) International publication number: WO 96/036298

(56) References cited:
- EP-A- 0 247 559
- EP-A- 0 611 556
- US-A- 3 894 540
- US-A- 4 580 568
- US-A- 5 158 548

## Description

### Field of the Invention

This invention relates to a stent delivery catheter system, such as the kind used in percutaneous transluminal coronary angioplasty (PTCA) procedures. More particularly, it relates to a stent delivery catheter employing a collapsible sheath which collapses during the retraction of a distal sheath during the release of a self-expanding or balloon expandable stent.

### Background of the Invention

In typical PTCA procedures, a guiding catheter is percutaneously introduced into the vascular system of a patient and advanced through the aorta until the distal end is in the ostium of the desired coronary artery. Using fluoroscopy, a guide wire is then advanced through the guiding catheter and across the site to be treated in the coronary artery. An over the wire (OTW) balloon catheter is advanced over the guide wire to the treatment site. The balloon is then expanded to reopen the artery. The OTW catheter may have a guide wire lumen which is as long as the catheter or it may be a rapid exchange catheter wherein the guide wire lumen is substantially shorter than the catheter. Alternatively, a fixed wire balloon catheter could be used. This device features a guide wire which is affixed to the catheter and cannot be removed.

To help prevent arterial closure, repair dissection, or prevent restenosis, a physician can implant an intravascular prosthesis, or a stent, for maintaining vascular patency inside the artery at the lesion. The stent may either be a self-expanding stent or a balloon expandable stent. For the latter type, the stent is often delivered on a balloon and the balloon is used to the expand the stent. The self-expanding stents may be made of shape memory materials such as NITINOL or constructed of regular metals but of a design which exhibits self expansion characteristics.

In certain known stem delivery catheters, a stent and an optional balloon are positioned at the distal end of the catheter, around a core lumen. The stent and balloon are held down and covered by a sheath or sleeve. When the distal portion is in its desired location of the targeted vessel the sheath or sleeve is retracted to expose the stent. After the sheath is removed, the stent is free to self-expand or be expanded with a balloon.

In a coronary stent deployment system which utilizes a retractable sheath one problem which is encountered is the interaction of the sheath and guide catheter upon retraction. The traditional way of dealing with this is to make the retractable sheath long enough so that it will be contained in the guide catheter at all times. This increases system profile, reduces flexibility and creates excess friction upon sheath retraction. The invention disclosed reduces the sheath length, maintains a reduced system profile and provides good flexibility.

In the prior art, E.P. Patent No. 611 556, issued to Advanced Cardiovascular Systems, Inc. discloses a stent delivery catheter system employing a retractable protective sheath according to the preamble of claim 1. The catheter has a plurality of lumens and a retracting member which retracts the protective sheath. When the protective sheath is retracted it slides back over the outer lumen.

### Summary of the Invention

The present invention provides an improved stent delivery system. The stent delivery system is characterized by the features of claim 1. During retraction of the distal sheath the collapsible sheath collapses upon itself, providing room for the distal sheath to retract unencumbered, thereby freeing the loaded stent. The inclusion of the collapsible sheath significantly reduces the required sheath length, maintains a reduced system profile, provides good flexibility and provides a protective covering to the wire pull back mechanism.

Other objects, features, embodiments and characteristics of the present invention, as well as the methods of operation and functions of the related elements of the structure, and the combination of parts and economics of manufacture, will become more apparent upon consideration of the following description with reference to the accompanying drawings, all of which form a part of this specification.

### Brief Description of the Figures

Figure 1 shows a side view of a catheter according to the invention having a loaded stent including a cross-sectional view of the distal portion thereof and a side view of the proximal end of a catheter according to the invention showing the manifold portion thereof.
Figure 2 shows a side view of a catheter according to the invention having a loaded self-expanding stent including a cross-sectional view of the distal portion thereof, wherein the loaded stent is shown as partially deployed, and a side view of the proximal end of a catheter according to the invention showing the manifold portion thereof.
Figure 3 shows a side view of a catheter according to the invention having a loaded stent including a cross-sectional view of the distal portion thereof, wherein the loaded stent is shown as fully deployed and a side view of the proximal end of a catheter according to the invention showing the manifold portion thereof.
Figure 4 shows a side view of a catheter according to an alternative embodiment of the invention having a loaded stent including a cross-sectional view of the distal portion thereof.
Figure 5 shows a side view of a catheter according to figure 4 having a loaded stent including a cross-sectional view of the distal portion thereof, wherein the loaded stent is shown as fully deployed.
Figure 6 shows a side view of a catheter according to an alternative embodiment of the invention having a loaded stent including a cross-sectional view of the distal portion thereof.
Figure 7 is a sectional view of the catheter thereof, taken along line 7-7 in Figure 6.
Figure 8 shows a side view of a catheter according to an alternative embodiment of the invention having a loaded stent including a cross-sectional view of the distal portion thereof.
Figure 9 shows a side view of a catheter according to an alternative embodiment of the invention having a loaded stent including a cross-sectional view of the distal portion thereof.
Figure 9a shows a partial exploded view of Fig. 9.
Figure 9b shows a partial exploded view of Fig. 9.
Figure 9c shows a partial exploded view of Fig. 9.

### Detailed Description of the Invention

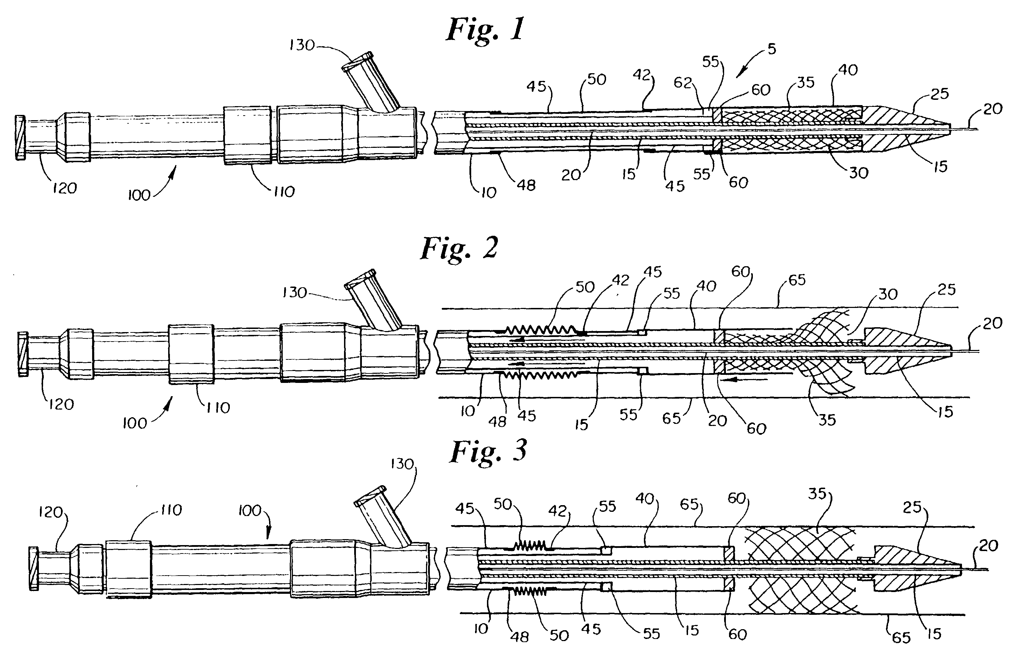

Figure 1 shows a cross-section of the distal portion of a specific embodiment of the stent delivery catheter, generally designated as 5, that is the subject of the present invention. The device generally comprises a proximal outer sheath 10 which covers the majority of the catheter 5 excluding a portion of the distal end of the catheter 5. This outer sheath 10 is characterized by a flexible tube which contains a pull wire lumen and a guide wire lumen. Preferably the outer sheath 10 is comprised of a high density polyethylene (HDPE), SURLYN, a combination of HDPE and low density polyethylene (LDPE) or NYLON material. The proximal outer sheath 10 encloses an optional guide wire lumen 15 which extends through and terminates with the distal tip 25 of the catheter 5. Preferably the guide wire lumen 15 encloses a guide wire 20 which aids in the navigation of the catheter 5 through the appropriate vessel. The guide wire lumen 15 is made of flexible, but incompressible construction such as a polymer encapsulated braid or coil. The flexibility of the braid/coil allows the catheter 5 to navigate through body lumens and the incompressibility of the braid/coil aids in maintaining the integrity of the catheter and aids in deployment accuracy when the sheath is being retracted during stent release. The braid/coil may be comprised of stainless steel or NITINOL, but preferably stainless steel encased in a polymer such as a polyimide, HDPE, TEFLON or urethane, but preferably polyimide and TEFLON.

Situated just proximal to the distal tip 25 is the portion 30 of catheter 5 around which the stent is concentrically carried. The stent 35 surrounds the guide wire lumen 25. The stent 35 is preferably a NITINOL™ or mesh self-expanding stent, but may also be a balloon expandable stent carried by an expansion balloon. Self-expanding and balloon expandable stents are well known in the art and require no further instruction.

The present invention further comprises a retractable distal sheath 40 which covers and contains the loaded stent 35. The retractable distal sheath 40 will hold a self-expanding stem in its reduced delivery configuration. The retractable distal sheath will merely contain a balloon expandable stent which was positioned over an expansion balloon. The distal sheath 40 is connected to a retracting member 45, or pull wire, which allows a physician to retract the distal sheath 40 from the proximal end of the catheter 5, thus releasing the stent 35 in the targeted area of the vessel. The retractable sheath 40 may be flexible or rigid, and is generally used to retain the stent 35 and protect the vessel wall during delivery. The distal sheath is preferably formed of a material which provides tensile strength, but is flexible, such as a braid, coil, a super elastic alloy, polymer, stainless steel or other similar composites. The retracting member 45 may be a rod, a cable, a tube which may also be used to transport fluids, a pull back wire, guide wire or the like, but is preferably a wire. In addition, the retracting member 45 may be tapered along its length to impart varying flexibility. Those skilled in the art will recognize other suitable materials and constructions may be employed to serve substantially the same function. The figures show two pull wires, but one is preferred. It should be understood that any desired number of pull wires could be utilized. The retracting member 45 extends longitudinally within the proximal outer sheath 10, optionally through a retracting member lumen (not shown), such as a HDPE, nylon or polyether block amide (PEBAX) tube. In one embodiment, the retracting member lumen extends longitudinally under the proximal outer sheath 10, and houses the pull back wire 45. The retracting member lumen that houses the pull back wire 45 may also carry fluid for purging air from the catheter 5.

The invention additionally comprises a collapsible sheath 50 situated between the proximal outer sheath 10 and the distal sheath 40. The collapsible sheath 50 covers the exposed area between the proximal outer 10 and the distal sheath 40, serving to protect the guide wire lumen 15 and the retracting member 45 in this area. The collapsible sheath 50 is adhered to the proximal end of the distal sheath 40 at point 42 and the distal end of the proximal outer 10 at point 48. These connections between components are preferably made using adhesives such as urethane or cyanoacrylate, and other suitable adhesives that are well known in the art. Connections between polymer components can also be made using other bonding techniques such as thermal welding, ultrasonic welding and the like.

The collapsible sheath 50 is manufactured to induce collapsibility by winding a coil around the collapsible sheath material, such as a tube of SURLYN. The coil winder controls the pitch or distance between adjacent wraps of wire. After the tube is wound, the tube is pressurized, causing the material to expand between the gaps in the wire and creating the pleats or creases which allow it to collapse. The coil is then removed producing the collapsible sheath 50 behind.

As the distal sheath 40 is retracted, the collapsible sheath 50 is forced back, collapsing upon itself into an accordion type configuration to give the distal sheath 40 room to retract. The collapsible sheath 50 is longer than the distal sheath 40 and is made from a highly flexible material such as SURLYN, PEBAX, or polyethylene, including both HDPE and LDPE, but preferably SURLYN. The distal sheath 40 and the collapsible sheath 50 may be two separate sheaths/components adhered to one another, or they may form one continuous sheath.

In the preferred embodiment, the distal sheath 40 is connected via a collar comprised of a short section of hypotube 55, configured as an annular ring, to the pull back wire 45. The proximal end of the distal sheath 40 is attached by adhesive or heat bond to the annular ring 55 and the distal end of the pull back wire 45 is connected, preferably brazed, to the inside of the annular ring 55. Although one pull back wire 45 is preferred, a plurality of pull back wires may be connected to the collar 55. The illustrative figures 1-5 enclosed herein utilize two pull back wires.

Proximal to the stent 35 is a stopper 60. The stopper 60 is preferably HDPE and is attached to the guide wire lumen 15, or whatever may comprise the rigid inner core, and is used to prevent the stent 35 from moving proximally when the distal sheath 40 is retracted.

Preferably, the catheter 5 further comprises an optional neck portion 62 located just proximal to the collar 55. This portion 62 is a slight reduction in diameter of the catheter 5 just behind the collar 55. The neck portion 62 aids in containing the collar 55 and supplies added leverage to the collar 55 as it retracts the distal sheath 40. It additionally aids in compressing the collapsible sheath 50 by providing an added brace for the collar 55 as the collar pushes back collapsing the collapsible sheath 50.

In an alternative embodiment a stiffening wire 60, preferably stainless steel but optionally NITINOL may also be incorporated longitudinally along the axis of the catheter 5 for extra stability and control.

In a fixed wire embodiment the guide wire lumen 15 may be replaced with just a guide wire, wherein the distal portion of the guide wire 20 is bonded to the distal tip 25.

The proximal portion of the catheter 5, as shown in Figures 1-3, comprises of a manifold system, generally designated 100, which includes a sliding member 110 slidably integrated between the distal and proximal end of the manifold. By retracting the sliding member 110 of the manifold 100, distal to proximal, the distal sheath 40 is retracted exposing the stent 35. The manifold 100 may further comprise a hydrating luer 130, which is preferably located on the distal end of the manifold 100 and is used to purge air from the catheter.

To prepare the stent delivery catheter 5 the stent 35 is compressed and loaded into the stent receiving portion 30 and covered by protective distal sheath 40. The distal sheath 40 remains covering the underlying stent 35 during the placement of the stent 35 by the delivery catheter 5 through the patient's vasculature. During the placement of the stent 35, the distal sheath 40 protects the patient's vasculature from the stent 35.

Figures 1-3 illustrate three stages of the deployment of a self-expanding stent 35 using the preferred embodiment of the catheter of the present invention. Figure 1 represents a loaded deployment catheter 5, with the stent 35 covered by the distal sheath 40 and the collapsible sheath 50 in its extended state. Figure 2 shows the stent 35 partially deployed, with the distal sheath retracted to cause the collapsible sheath to partially collapse. In the preferred embodiment the pull wire is attached to sliding member 110, which is used to retract sheath 40. As the sliding member 110 is pulled back, the distal sheath 40 begins to retract. The stent is prevented from moving proximally with the sheath by the stopper and therefore, the stent 35 begins to release and expand while the collapsible sheath 50 begins to collapse upon itself in an accordion fashion. Since the distal sheath 40 does not slide back over the proximal outer sheath, but rather the collapsible sheath 50 collapses in place, the profile of the catheter 5 remains nearly the same. Figure 3 shows the stent fully released. At this point the distal sheath 40 is fully retracted and the collapsible sheath 50 is compressed releasing the stent 35 to allow it to self-expand against the vessel wall 65. After the stent 35 is expanded and in place, the catheter 5 is withdrawn. It should be understood that a balloon expandable stent could also be utilized by arranging the stent around an optional placement balloon (not shown). Once the sheath 40 is fully retracted the placement balloon would be inflated through its inflation lumen (not shown) to deploy the stent.

Preferably the stent 35 is self expanding, such as a NITINOL™ stent, or it may be expanded by means of an internal balloon positioned under the stent 35 on the distal end of the inner core 40. Those skilled in the art will recognize other suitable materials and constructions which may be employed to serve substantially the same function.

The collapsible sheath is formed such that upon retraction of the distal sheath 40 the collapsible sheath 50 is compressed to a state approximately 1/5 of its longitudinally expanded state. The collapsible sheath 50 provides covering of the wire mechanism, eliminates the relative motion of the proximal edge of the distal sheath 40 and reduces the friction involved in retraction the distal sheath 40. Unlike known retractable systems, the distal sheath does not retract over or under the proximal outer, which results in an increase in the profile of the catheter, an increase in friction as the distal sheath resists being pulled back over the proximal outer and a higher likelihood of hang ups due to the faulty engagement between the proximal end of the distal sheath and the guide catheter or vessel. In the present invention the collapsible sheath 50 compresses thereby providing space for the distal sheath 40 to retract without any encumbrances.

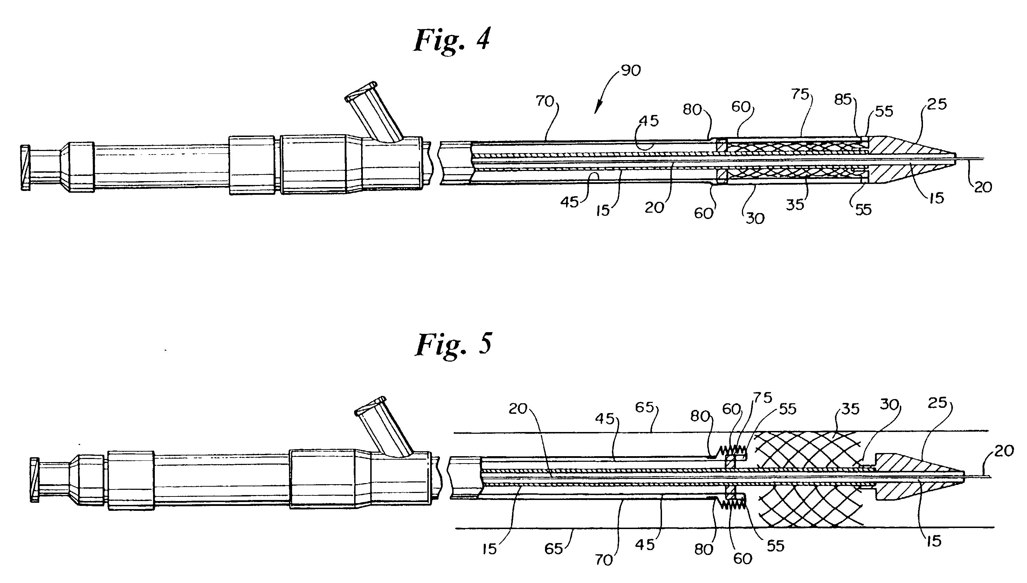

Figures 4 and 5 illustrate an alternative embodiment of the present invention. In this case the proximal outer sheath 70 extends distally over the catheter, generally designated 90, up to a position in close proximity with the stopper 60 and the collapsible sheath 75 performs as the distal sheath. The distal end of the proximal outer sheath 70 is adhered to the proximal end of the collapsible sheath 75 at point 80. In this embodiment the collar 55 is connected to collapsible sheath 75 at the distal end at point 85. As the pull back wire 45 is drawn proximally, the collapsible sheath 75 is retracted, collapsing upon itself, and begins to release. As discussed earlier, stopper 60 prevents the stent from moving proximally with the retracting sheath 75. Figure 5 illustrates the fully retracted collapsible sheath 75 and the release of the stent 35 to its fully expanded position urging against the inner wall of the vessel 65.

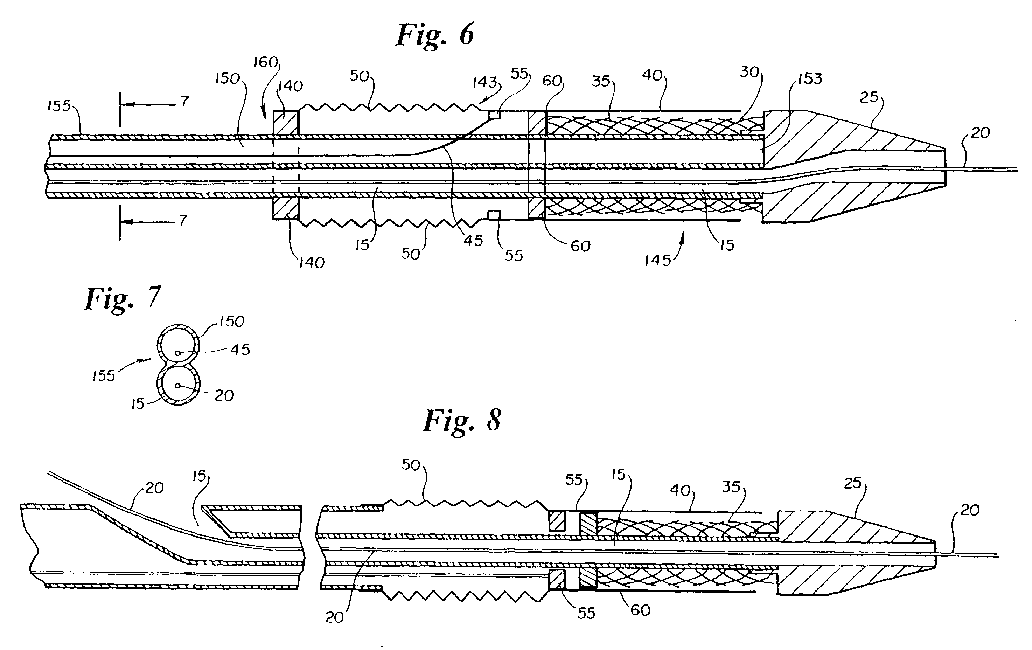

Figure 6 discloses an alternative embodiment of the present invention. In this case the stent delivery system is generally designated 145 and the catheter 155 is comprised of a guide wire lumen 15 and a pull back lumen 150. The pull back lumen is axially connected to the guide wire lumen, traveling along the length of the guide wire lumen 15 up to the distal tip 25 at point 153, as the guide wire lumen continues through the distal tip 25. Figure 7 illustrates the configuration of the catheter 155 from a cross-section perspective along lines 7-7 in Figure 6. A stent 35 may be concentrically arranged around the catheter 15 near the distal end on the stent receiving portion 30. The device further comprises a retractable distal sheath 40 surrounding at least a portion of the stent 35. Figure 6 shows the retractable distal sheath 40 partly retracted. The proximal end of the retractable distal sheath 40 is attached to the collapsible sheath 50 at point 143. The collapsible sheath 50 is concentrically arranged around the catheter 155 and is shown in Figure 6 as partially collapsed. The proximal end of the collapsible sheath 50 is connected, preferably adhered, to a fixed anchoring device 140, preferably an annular collar, which is affixed to the catheter 155 at point 160. The fixed anchoring device 140 stabilizes the proximal end of the collapsible sheath 50 allowing it to collapse upon itself during retraction of the distal sheath 40. The pull back wire 45 travels, proximal to distal, through the pull back lumen 150 and exits through an axial slit (not shown) in the surface of the pull back lumen 150. The distal end of the pull back wire is attached to annular ring 55, which is in turn attached to the retractable distal sheath 40. During the application of the device the pull back wire 45 is retracted, sliding proximally through the axial slit in the pull back lumen, proximally retracting the distal sheath 40 causing the collapsible sheath 50 to collapse, freeing the stent 35 for delivery. The stopper 60 prevents the stent from moving proximally with the retracting sheath 75.

Figure 8 illustrates a rapid exchange embodiment of the invention. The distal end of the catheter is structured and functions in the same fashion as that of device shown in Figure 1. The overall length of the catheter is approximately 135 cm, while the length of the guide wire lumen 15 is between approximately 5 cm to 35 cm from the distal tip 25 to a point where the guide wire lumen 15 and the guide wire 20 exit the catheter.

It should be understand that other mechanical methods of retracting the pull back wire, besides the manifold apparatus disclosed herein, may be employed.

It should also be understood that the retractable distal sheath 40 and the collapsible sheath 50 may comprise one continuous sheath, wherein the preform creases or pleats are incorporated only into the intended collapsible portion.

The design disclosed herein also aids in flushing the catheter. Since the catheter essentially is sealed to the distal tip 25 and has only one opening in the distal segment, that being the end portion of the guide wire lumen 15 at the distal tip 25, flushing is made easier and more efficient.

The present invention may be incorporated into both of the two basic types of catheters used in combination with a guide wire, commonly referred to as over-the-wire (OTW) catheters and rapid-exchange (RX) catheters. The construction and use of both over-the-wire and rapid-exchange catheters are well known in the art. The usable length of the delivery catheter is approximately 135 cm. For a rapid exchange catheter the distance from where the guide wire accesses the guide wire lumen to the distal tip will be approximately 5 cm to 35 cm.

The key features of the longitudinally collapsible sheath include, without limitation: low profile both proximally and distally when extended, relatively thin walls for low profile and large interior lumens, efficient packing upon collapse, flexible, pushable and trackable.

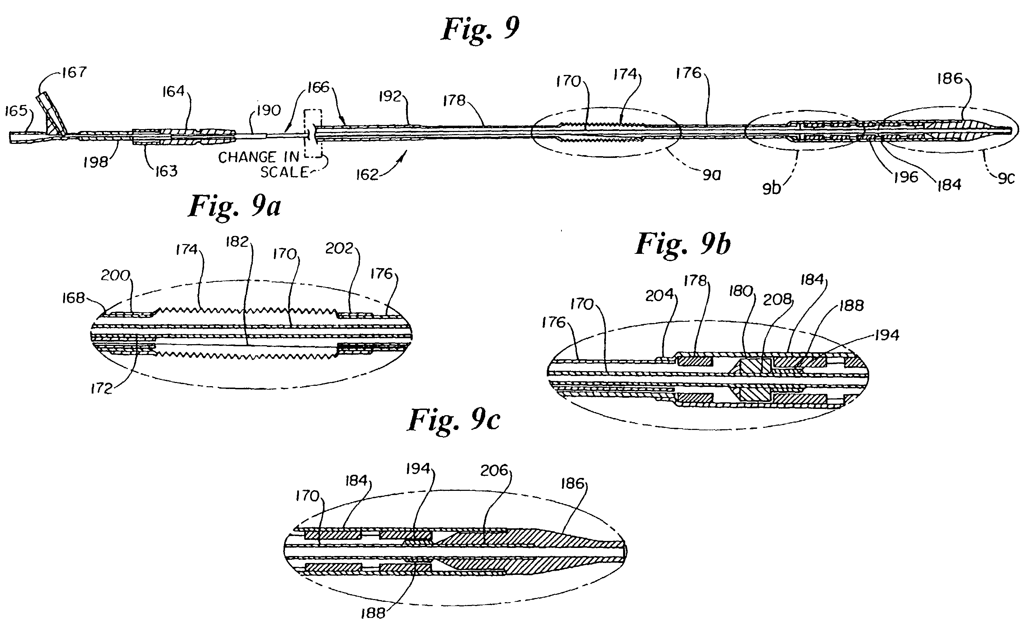

A further embodiment of the invention is shown in Figures 9, 9a, 9b and 9c. This catheter, generally designated 162, is similar to the above embodiments in that it focuses on a retractable sheath portion and an accordion portion, and it comprises many of the same materials and elements, which function in substantially the same manner.

As shown in Fig. 9, the catheter 162 includes a hand manifold, generally designated 164, which comprises a handle 161, a sheath actuator (sliding member) 163. a safety lock 198, which secures the sheath actuator 164 in place and is preferably made of polyethylene, a guide wire inlet 165 for controlling the guide wire from the proximal end, and a hydrating luer 167, for flushing. The manifold 164 also comprises a strain relief tubing 190 which is connected to the distal end of the handle. The proximal outer shaft 166 extends distally through the strain relief tubing 190. The strain relief tubing 190 protects the proximal outer shaft 166 at the shaft's 166 joining with the manifold 164. Such protection is needed because of the distinct size difference between the manifold 164 and the proximal outer shaft 166.

The catheter 162 further comprises a proximal outer shaft, or proximal outer, 166, preferably made of a polyimide braid, a distal outer shaft 168, preferably made of polyethylene, connected to the proximal outer shaft 166 at an adhesive point 192, preferably by means of a urethane adhesive. The distal outer shaft 168 is similarly connected to a collapsible sheath/shaft 174, which is collapsible in the longitudinal direction in an accordion manner at a second adhesive point 200 (preferably using a urethane adhesive) and which is most preferably made of HDPE, best shown in Fig. 9a. The collapsible shaft 174 is in turn connected to a proximal sheath, or spacer sheath, 176, which is preferably made of polyethylene, at a third adhesive point 202 (preferably using a urethane adhesive). The distal end of the proximal sheath 176 is adhered at point 204 (preferably using a urethane adhesive) to the proximal end of a retractable distal sheath, or deployment sheath, 184, which is preferably made of polyethylene, which encloses a loaded stent 196, preferably a NITINOL self expanding stent.

The catheter 162 also further comprises an internal guide wire lumen 170, preferably made of a polyimide braid, which may house a guide wire and which extends within the catheter 162 from the guide wire inlet 165 to and through a distal tip 186, which is preferably made of polyethylene, at the distal end of the catheter 162, to which it is connected to the distal tip 186, best seen in Fig. 9c, preferably using a urethane adhesive 206. The deployment sheath 184 overlaps the distal tip 186 in a manner which provides the smoothest profile possible. In Fig. 9c, this "smooth profile" is achieved by a notch in the outer surface of the distal tip 186 in which the distal end of the deployment sheath abuts.

Also housed within the catheter is a pull (back) wire lumen 172, preferably made of polyethylene, which in turn houses the pull (back) wire 182, which is preferably stainless steel. The pull wire lumen 172, best seen in Figs. 9a and b, is optional and can be adhered and separate from the outer wall of the catheter 162, or can share common inner surfaces with the outer wall of the catheter 162. The pull wire lumen 172 extends from a point just distal to the sheath actuator 163, through the catheter 162, to a point at the distal end of the proximal sheath 176, best seen in Fig. 9b. As seen in Fig. 9a, the pull wire lumen may discontinue and recontinue when within the collapsible shaft 174. This is to allow the shaft 174 room to collapse when the deployment sheath 184 is retracted.

The pull wire 182 is housed within the pull wire lumen 172. The proximal end of the pull wire 182 is attached to the sheath actuator 163 and the distal end of the pull wire 182 is connected to a pull collar (annular ring) 178, preferably made of stainless steel, which, as seen in Fig. 9b, is connected, preferably adhered, to the inner wall of the proximal end of the deployment sheath 184. This collar, as in the above embodiments, can be positioned anywhere along the catheter distal to the collapsible portion, as long as it is connected so that the sheath covering the stent is retracted. As in the other embodiments, when the sheath actuator 163 is retracted, the deployment sheath is similarly retracted, pushing back the proximal sheath 176, which in turn applies reward pressure on the collapsible shaft, which collapses onto itself, resulting in the release of a stent 196.

Fig. 9b further illustrates a stopper/bumper 180, preferably made of polyethylene, positioned just proximal to the stent 196 used to prevent the stent 196 from slipping proximally during the retraction of the deployment sheath 184. The bumper is preferably adhered to the guide wire lumen 170 using a urethane adhesive 208 just distal to the pull collar 178 within the deployment sheath 184.

Figs. 9b and 9c also illustrate a pair of optional markerbands 188 positioned at either end of the loaded stent 196 to provide the physician means to accurately position the stent within the vascular lumen at the targeted problem area where the stent is to be deployed. Any number, or size, of markerbands may be incorporated, as long as it aids the physician in accurately positioning the stent. Preferably, there are two, positioned as seen in Figs. 9b and 9c, and are adhered to the guide wire lumen at the proximal and distal ends 194 of the stent 196, preferably using an cyanoacrylate adhesive.

The distal outer shaft 168, preferably made of polyethylene, and the proximal sheath 176, have reduced diameters as compared to the proximal outer shaft 166 and the deployment sheath 184, respectively, in order to reduce the profile of the collapsible shaft 174. As can be seen in Figs. 9, 9a and 9b, the outside of the distal end of the proximal outer shaft 166 is adhered to the inside of the proximal end of the distal outer shaft 168. Just distal to this connection, the diameter of the distal outer shaft 168 is decreased. Similarly, the outside of the distal end of the distal outer shaft 168 is adhered to the inside of the proximal end of the collapsible shaft 174. The inside of the distal end of the collapsible shaft 174, in turn, is adhered to the outside of the proximal end of the proximal sheath 176, which has approximately the same diameter of the distal outer shaft 168. The outside of the distal end of the proximal sheath 176, finally, is adhered to the inside of the proximal end of the deployment sheath, which is notched down so as to be compatible with the outer diameter of the distal end of the proximal sheath 176 and to aid in maintaining the position of the pull collar 178 during retraction, best seen in Fig. 9b. This type of construction reduces the profile of the collapsible shaft 174 when the shaft is collapsed. The distal outer shaft 168 and the proximal sheath 176 could be removed so that the distal end of the proximal outer shaft 166 is extended and is adhered to the proximal end of the collapsible shaft 174, while the proximal end of the deployment sheath 184 is extended and is adhered to the distal end of the collapsible shaft 174, but 4the overall provide would be compromised.

It should be further noted that the collapsible portion of the catheter can be located anywhere along the catheter distal to the manifold and proximal to the retractable portion in the above embodiments. The collapsible portion can also be any length, as long as it is longer than the length of the loaded stent so as to allow for clean deployment of the stent.

Any part of the above sheaths of the catheter distal to the collapsible portion is considered the retractable portion. The retractable portion proximal to the pull collar and the shaft/sheath portions proximal to the collapsible portion must be rigid enough to resist collapsing upon itself during the sheath retracting process. The guide wire lumen also must be rigid enough to resist the reward pressure during the retraction of the retractable portion.

The collapsible shaft is constructed so as to collapse upon retraction of the retractable portion. The following are descriptions of example methods used in creating a polyethylene collapsible sheath and a SURLYN collapsible sheath:

### Example 1

### Process for Polyethylene (PE) Collapsible Shaft Forming

### Materials needed:

1. PE tubing 0.041/0.046 (inches) inner/outer diameter
2. Mandrel stock 0.040 (inches)
3. Coil winder
4. Blade with 0.003 radius edge (inches).
5. Rubber Gloves

### Protocol:

- Step 1.: Place the PE tubing onto the mandrel and into coilwinder.
- Step 2.: Turn coilwinder on at speed 4, with a .020 pitch and set blade at 35 degree angle.
- Step 3.: Set blade down on PE tube and plow, or contour, the PE tube. The resulting PE tube will have a small indent circumferentially around the tube, preferably in a helical fashion.
- Step 4.: Hold the PE tube on outside of indent and push together so tube will accordion, preferably with rubber gloves.
- Step 5.: Leave accordion tube on mandrel for annealing process. Anneal tube in an oven at 70 degrees C. for a minimum of 4 hours.

The resulting shaft is then prepared to be incorporated into the catheter.

### Example 2:

### Process for SURLYN Collapsible Shaft Forming

### Materials needed:

1. Razor blades
2. Balloon Mold (preferably 1.5mm)
3. Spring
4. Cold water bath and hot water bath (80° C)
5. Mandrel (preferably TEFLON coated)
6. SURLYN sleeve (blank)
7. Alcohol (99%)
8. Grooved accordion mold
9. Air pressure station (preferably 95-100 PST)

### Protocol:

- Step 1.: Load the blank tubing into the mold using the following method:
Load spring into the balloon mold (note: no spring is required with the grooved accordion mold). Insert tubing blank into mold until approximately ½ protrudes from proximal end. Place balloon mold into holding bracket. Insert end of tubing blank into the Y adapter connector and tighten. Pressurize tube.
- Step 2.: Mold the accordion assembly using the following method:
Lower mold into hot water bath up to the Y adapter and then raise until top edge of mold reaches surface of water. Hold mold in hot water for 25-30 seconds. Lift mold out of water bath and insert into cold water bath for 3-5 seconds. Remove mold from water and close air stopcock. Open Y adapter valve and remove tubing. Remove balloon mold from bracket. Open mold and pull out tubing with spring (if present). Trim proximal end of tubing to 5mm and remove spring (if present).
- Step 3.: Heat set the folds of the accordion assembly using the following method:
Slide accordion assembly over mandrel. Slide black sleeve over mandrel and under shaft of accordion assembly up to the folds. Push both ends of the folds together from each end and place a clamp onto the sleeve to hold it in place. Dip assembly into hot water bath a maximum of 2mm past folds and hold for 10-15 seconds (note: ensure folds are submerged in the water). Remove and dip mandrel into cold water bath for 2-5 seconds. Remove from cold water bath and slide back sleeve and clamp. Slide accordion assembly off mandrel. Rinse with alcohol and dry with nitrogen.

The resulting shaft is then prepared to be incorporated into the catheter.

It should also be noted that the part of the retractable portion which covers the loaded stent can also be contoured to increase its flexibility, preferably in a helical fashion.

The above disclosure is intended to be illustrative and not exhaustive.

These examples and description will suggest many variations and alternatives to one of ordinary skill in this art. All these alternatives and variations are intended to be included within the scope of the attached claims. Those familiar with the art may recognize other equivalents to the specific embodiments described herein which equivalents are also intended to be encompassed by the claims attached hereto.

## Claims

1. A stent delivery system comprising:
- a catheter (5) with a distal end and a proximal end;
- a stent receiving portion (30) adapted to receive a stent (35) near the distal end of the catheter;
- a retractable distal sheath (40, 75, 176, 184) having a proximal end and a distal end, and surrounding at least a portion of the catheter;
- a pull back means (45) having a distal end connected to the retractable distal sheath (40) for retraction of the retractable distal sheath (40);
**characterized in that** the catheter includes
- a collapsible sheath (50, 75, 174) concentrically arranged around the catheter, the collapsible sheath being capable of collapsing upon itself in the longitudinal direction, wherein upon proximal retraction of the pull back means (45) the distal retractable sheath (40, 75, 176, 184) is retracted, causing the collapsible sheath (50, 75, 174) to collapse axially upon itself.

2. The stent delivery system as in claim 1, wherein the collapsible sheath is positioned proximal to the retractable sheath, the stent delivery system further comprising a proximal outer sheath (10) positioned on the catheter proximal to the collapsible sheath (50), wherein the collapsible sheath (50) has a distal end and a proximal end, the distal end being adhered to the retractable distal sheath (40) and the proximal end being adhered to the proximal outer sheath (10).

3. The stent delivery system as in claim 2, further comprising a self-expanding stent (35) loaded on the distal end of the catheter, wherein the stent (35) is held in its collapsed state by the retractable distal sheath (40) and when the pull back means (45) is retracted proximally the distal sheath (40) is retracted, causing the collapsible sheath (50) to collapse and freeing the stent (35) for delivery.

4. A stent delivery system as in claim 3, wherein the pull back means (45) comprises a pull back wire (45).

5. The stent delivery system as in claim 4, wherein the pull back means (45) further comprises an annular collar (55) surrounding the catheter and longitudinally movable along the catheter, wherein the proximal end of the retractable distal sheath (40) is connected to the annular collar (55) and the distal end of the pull back wire (45) is connected to the annular collar.

6. The stent delivery system as in claim 5, further comprising a stopper (60) positioned on the catheter to prevent the stent from moving proximally as the distal sheath is retracted to expose the stent (35).

7. A stent delivery system as in claim 6, wherein the catheter comprises a guide wire lumen (15), the guide wire lumen being incompressible and flexible.

8. A stent delivery system as in claim 6, wherein the catheter comprises a guide wire (20), the guide wire being bonded to the distal end.

9. The stent delivery system as in claim 7, further comprising a pull back wire lumen which partially encloses the pull back wire (45) and is at least partially covered by the proximal outer sheath (10).

10. The stent delivery system as in claim 8, further comprising a stiffening wire positioned longitudinally along the catheter.

11. The stent delivery system as in claim 7, wherein the guide wire lumen (15) is a polymer encapsulated braid.

12. The stent delivery system as in claim 7, wherein the guide wire lumen (15) is a polymer encapsulated coil.

13. The stent delivery system as in claim 11, further comprising a guide wire (20) enclosed within the guide wire lumen.

14. The stent delivery system as in claim 13, comprising a plurality of pull back wires (45) attached to the annular collar (55).

15. The stent delivery system as in claim 14, wherein the retractable distal sheath (40) further includes a neck portion (62) just proximal to the connection between the retractable distal sheath (40) and the annular collar (55), which frictionally engages the annular collar and aids in retraction of the distal sheath (40).

16. The stent delivery system as in claim 14, wherein the proximal outer sheath (10) comprises a high density polyethylene.

17. The stent delivery system as in claim 7, wherein the stent delivery system is an over-the wire catheter.

18. The stent delivery system as in claim 7, wherein the guide wire lumen (15) is shorter than the catheter.

19. The stent delivery system of claim 18, wherein the catheter is 135 cm and the guide wire lumen (15) is between 5 cm and 35 cm.

20. The stent delivery system as in claim 2, further comprising a balloon disposed under the stent, whereby after the retractable distal sheath (40) is retracted the stent (35) is expandable by inflating the balloon.

21. The stent delivery system as in claim 1, further comprising:
- a distal tip (25) arranged distal to the stent receiving portion; the collapsible sheath being
- the retractable distal sheath (75) and surrounding the stent receiving portion (30).

22. The stent delivery system as in claim 21, further comprising a proximal outer sheath (70) positioned on the catheter proximal to the retractable distal sheath (75), wherein the retractable distal sheath (75) comprises a proximal end, the proximal end being adhered to the proximal outer sheath (70).

23. The stent delivery system as in claim 22, further comprising a self-expanding stent (35) loaded on the distal end of the catheter, wherein the stent (35) is held in its collapsed state by the retractable distal sheath (75) and when the pull back means (45) is retracted proximally the retractable distal sheath (75) is retracted, causing the retractable distal sheath to collapse and freeing the stent (35) for delivery.

24. The stent delivery system as in claim 23, further comprising an annular collar (55), wherein the proximal end of the retractable distal sheath (75) and the distal end of the pull back means (45) are connected to the collar.

25. The stent delivery system as in claim 24, further comprising a stopper (60) positioned to prevent the stent from moving proximally as the retractable distal sheath (75) is retracted to expose the stent (35).

26. A stent deliver system as in claim 25, wherein the pull back means (45) is a pull back wire.

27. A stent delivery system as in claim 26, wherein the catheter comprises a guide wire lumen (15), the guide wire being incompressible and flexible.

28. A stent delivery system as in claim 26, wherein the catheter comprises a guide wire (45), the guide wire being bonded to the distal tip (25).

29. The stent delivery system as in claim 27, further comprising a pull back wire lumen which partially encloses the pull back wire (45) and is at least partially covered by the proximal outer sheath (70).

30. The stent delivery system as in claim 28, further comprising a stiffening wire positioned longitudinally along the catheter.

31. The stent delivery system as in claim 27, wherein the guide wire is a polymer encapsulated braid.

32. The stent delivery system as in claim 27, wherein the guide wire lumen (15) is a polymer encapsulated coil.

33. The stent delivery system as in claim 31, further comprising a guide wire (45) enclosed within the guide wire lumen (15).

34. The stent delivery system as in claim 33 comprising a plurality of pull back wires.

35. The stent delivery system as in claim 34, further comprising a neck portion situated in the retractable distal sheath (75) just proximal to the attachment between the retractable distal sheath (75) and the annular collar.

36. The stent delivery system as in claim 34, wherein the proximal outer sheath (70) comprises a high density polyethylene.

37. The stent delivery system as in claim 27, wherein the stent delivery system is an over-the wire catheter.

38. The stent delivery system as in claim 27, wherein the catheter is 135 cm and the guide wire lumen (15) is between 5 cm and 35 cm.

39. The stent delivery system as in claim 21, further comprising a balloon disposed under the stent within the stent receiving portion (30), whereby after the retractable distal sheath (75) is retracted the stent (35) is expanded by inflating the balloon.

40. The stent delivery system as in claim 1, further comprising:
- a guide wire lumen (15) and a pull back lumen (150);
- a stent (35) concentrically arranged around the catheter near the distal end; and an anchoring device (140) fixedly attached to the catheter;
- wherein a portion of the pull back means (45) is housed in the pull back lumen, and wherein the collapsible sheath (50) has proximal and distal ends and is concentrically arranged around the catheter, the proximal end of the collapsible sheath (50) being attached to the anchoring device (140) and the distal end of the collapsible sheath (50) being attached to the proximal end of the retractable distal sheath (40).

41. The stent delivery system as in claim 1, further comprising proximal outer shaft (166) positioned on the catheter proximal to the collapsible sheath (174), wherein the collapsible sheath is positioned on the catheter proximal to the retractable distal sheath.

42. The stent delivery system as in claim 41, wherein the retractable distal sheath comprises a proximal sheath (176) and a deployment sheath (184), both the proximal sheath and the deployment sheath being distal to the collapsible sheath (174) and the proximal sheath being proximal to the deployment sheath, and wherein the proximal sheath is connected to the collapsible sheath (174) and the deployment sheath.

43. The stent delivery system as in claim 42, further comprising a distal outer shaft (168), the distal outer shaft being positioned on the catheter between, and connected to, the proximal outer shaft and the collapsible sheath (174).

44. The stent delivery system as in claim 43, further comprising a guide wire lumen (170) internally extending from the proximal end of the catheter to the distal end of the catheter.

45. The stent delivery system as in claim 44, further comprising a distal tip (186) positioned and attached to the distal end of the guide wire lumen.

46. The stent delivery system as in claim 45, wherein the pull back means (182) is a pull wire, and further comprising a pull collar (178) connected to the retractable distal sheath, the distal end of the pull wire being connected to the pull collar.

47. The stent delivery system as in claim 46, further comprising a manifold (164) positioned at the proximal end of the catheter, the manifold comprising a sheath actuator (163), wherein the proximal end of the pull wire (182) is connected thereto.

48. The stent delivery system as in claim 47, further comprising a pull wire lumen (172) internally extending from just distal to the manifold (164) to just proximal to the pull collar (178).

49. The stent delivery system as in claim 48, wherein the pull collar (178) is attached to the proximal end of the deployment sheath.

50. The stent delivery system as in claim 49, wherein the pull wire lumen (172) is discontinued and recontinued at the proximal end and the distal end of the collapsible sheath (174), respectively.

51. The stent delivery system as in claim 50, further comprising a self expanding stent (35) loaded on the distal end of the guide wire lumen (170), just proximal to the distal tip, under the deployment sheath, wherein the stent (35) is held in its collapsed state by the deployment sheath (184) and when the pull back wire (172) is retracted proximally, the retractable distal sheath is retracted, causing the collapsible sheath (174) to collapse and freeing the stent (35) for delivery.

52. The stent delivery system as in claim 51, further comprising a bumper (180) attached to the guide wire lumen (170) just proximal to the loaded stent (35) to prevent the stent from moving proximally as the deployment sheath is retracted to expose the stent.

53. The stent delivery system as in claim 52, further comprising marker bands (194) attached to the guide wire lumen (170) in close proximity to the loaded stent (35) so as to allow the user to accurately position the stent within a targeted vascular lumen.

## Patentansprüche

1. Stent-Anbringungssystem, das folgendes aufweist:
- einen Katheter (5) mit einem distalen Ende und einem proximalen Ende;
- einen Stent-Aufnahmeabschnitt (30), der zur Aufnahme eines Stents (35) nahe dem distalen Ende des Katheters angepaßt ist,
- eine zurückziehbare distale Hülle (40, 75, 176, 184) mit einem proximalen Ende und einem distalen Ende, die mindestens einen Abschnitt des Katheters umgibt;
- eine Zurückzieheinrichtung (45), die ein distales Ende besitzt, das mit der zurückziehbaren distalen Hülle (40) zum Zurückziehen der zurückziehbaren distalen Hülle (40) verbunden ist;
**dadurch gekennzeichnet, daß** der Katheter folgendes umfaßt:
- eine zusammenlegbare Hülle (50, 75, 174), die konzentrisch um den Katheter angeordnet ist, wobei die zusammenlegbare Hülle in longitudinaler Richtung auf sich selbst zusammenlegbar ist, wobei auf ein proximales Zurückziehen der Zurückzieheinrichtung (45) die distale zurückziehbare Hülle (40, 75, 176, 184) zurückgezogen wird, wodurch die zusammenlegbare Hülle (50, 75, 174) sich axial auf sich selbst zusammenlegt.

2. Stent-Anbringungssystem nach Anspruch 1, wobei die zusammenlegbare Hülle proximal von der zurückziehbaren Hülle positioniert ist, das Stent-Anbringungssystem zusätzlich eine proximale äußere Hülle (10) aufweist, die auf dem Katheter proximal von der zusammenlegbaren Hülle (50) positioniert ist, wobei die zusammenlegbare Hülle (50) ein distales Ende und ein proximales Ende besitzt, das distale Ende an der zurückziehbaren distalen Hülle (40) und das proximale Ende an der proximalen äußeren Hülle (10) angeklebt sind.

3. Stent-Anbringungssystem nach Anspruch 2, das zusätzlich einen selbstausdehnenden Stent (35) aufweist, der auf dem distalen Ende des Katheters geladen ist, wobei der Stent (35) in seinem zusammengelegten Zustand durch die zurückziehbare distale Hülle (40) gehalten ist und, wenn die Zurückzieheinrichtung (45) proximal zurückgezogen ist, die distale Hülle (40) zurückgezogen ist, wodurch die zusammenlegbare Hülle (50) sich zusammenlegt und den Stent zur Anbringung befreit.

4. Das Stent-Anbringungssystem nach Anspruch 3, bei dem die Zurückzieheinrichtung (45) einen Zurückziehdraht (45) aufweist.

5. Das Stent-Anbringungssystem nach Anspruch 4, bei dem die Zurückzieheinrichtung (45) zusätzlich einen ringförmigen Bund (45) aufweist, der den Katheter umgibt und longitudinal entlang dem Katheter verschiebbar ist, wobei das proximale Ende an der zurückziehbaren distalen Hülle (40) mit dem ringförmigen Bund (55) und das proximale Ende des Zurückziehdrahtes (45) mit dem ringförmigen Bund verbunden ist.

6. Stent-Anbringungssystem nach Anspruch 5, das zusätzlich einen Anschlag (60) aufweist, der auf dem Katheter positioniert ist, um den Stent an einer proximalen Bewegung zu hindern, wenn die distale Hülle zur Freigabe des Stents (35) zurückgezogen ist.

7. Stent-Anbringungssystem nach Anspruch 6, bei dem der Katheter eine Führungsdrahtöffnung (15) aufweist, wobei die Führungsdrahtöffnung inkompressibel und flexibel ist.

8. Stent-Anbringungssystem nach Anspruch 6, bei dem der Katheter einen Führungsdraht (20) aufweist, wobei der Führungsdraht an das distale Ende angebunden ist.

9. Stent-Anbringungssystem nach Anspruch 7, das zusätzlich eine Zurückziehdrahtöffnung besitzt, die den Zurückziehdraht (45) teilweise umgibt und mindestens teilweise durch die proximale äußere Hülle (10) bedeckt ist.

10. Stent-Anbringungssystem nach Anspruch 8, das zusätzlich einen versteiften Draht aufweist, der longitudinal entlang dem Katheter angeordnet ist.

11. Stent-Anbringungssystem nach Anspruch 7, bei dem die Führungsdrahtöffnung (15) eine polymergekapselte Litze ist.

12. Das Stent-Anbringungssystem aus Anspruch 7, bei dem die Führungsdrahtöffnung (15) eine polymergekapselte Spule ist.

13. Stent-Anbringungssystem aus Anspruch 11, das zusätzlich einen Führungsdraht (20) aufweist, der in die Führungsdrahtöffnung eingeschlossen ist.

14. Stent-Anbringungssystem nach Anspruch 13, mit einer Vielzahl von Zurückziehdrähten (45), die an den ringförmigen Bund (55) befestigt sind.

15. Stent-Anbringungssystem nach Anspruch 14, bei dem die zurückziehbare distale Hülle (40) zusätzlich einen Nackenabschnitt (62) direkt proximal von der Verbindung zwischen der zurückziehbaren distalen Hülle (40) und dem ringförmigen Bund (55) aufweist, der reibend an dem ringförmigen Bund angreift und ein Zurückziehen der distalen Hülle (40) unterstützt.

16. Das Stent-Anbringungssystem nach Anspruch 14, wobei die proximale äußere Hülle (10) ein hoch dichtes Polyethylen aufweist

17. Stent-Anbringungssystem nach Anspruch 7, wobei das Stent-Anbringungssystem ein über-dem-Draht Katheter ist.

18. Stent-Anbringungssystem nach Anspruch 7, bei dem die Führungsdrahtöffnung (15) kürzer als der Katheter ist.

19. Stent-Anbringungssystem nach Anspruch 18, bei dem der Katheter 135 cm und die Führungsdrahtöffnung (15) zwischen 5 cm und 35 cm ist.

20. Stent-Anbringungssystem nach Anspruch 2, das zusätzlich einen unter dem Stent angeordneten Ballon aufweist, wobei nach dem die zurückziehbare distale Hülle (40) zurückgezogen ist, der Stent (35) durch Aufblasen des Ballons ausdehnbar ist.

21. Stent-Anbringungssystem nach Anspruch 1, das zusätzlich aufweist:
- eine distale Spitze (25), die distal zu dem Stent-Aufnahmeabschnitt angeordnet ist,
- wobei die zusammenlegbare Hülle die zurückziehbare distale Hülle (75) ist und den Stent-Aufnahmeabschnitt (30) umgibt.

22. Stent-Anbringungssystem nach Anspruch 21, das zusätzlich eine proximale äußere Hülle (70) aufweist, die auf den Katheter proximal zu der zurückziehbaren distalen Hülle (75) positioniert ist, wobei die zurückziehbare distale Hülle (75) ein proximales Ende besitzt, das an die proximale äußere Hülle (70) geklebt ist.

23. Stent-Anbringungssystem aus Anspruch 22, das zusätzlich einen selbstausdehnenden Stent aufweist, der auf dem distalen Ende des Katheters geladen ist, wobei der Stent (35) in seinem zusammengelegten Zustand durch die zurückziehbare distale Hülle (75) gehalten ist und, wenn die Zurückzieheinrichtung (45) proximal zurückgezogen ist, ist die zurückziehbare distale Hülle (75) zurückgezogen, wodurch die zurückziehbare distale Hülle zusammengelegt wird und den Stent (35) zur Anbringung befreit.

24. Stent-Anbringungssystem nach Anspruch 23, das zusätzlich einen ringförmigen Bund aufweist, wobei das proximale Ende der zurückziehbaren distalen Hülle (75) und das distale Ende der Zurückzieheinrichtung (45) mit dem Bund verbunden sind.

25. Stent-Anbringungssystem nach Anspruch 24, das zusätzlich einen Anschlag (60) aufweist, der positioniert ist, um den Stent an einer proximalen Bewegung zu hindern, wenn die zurückziehbare distale Hülle (75) zurückgezogen ist, um den Stent (35) freizugeben.

26. Stent-Anbringungssystem nach Anspruch 25, wobei die Zurückzieheinrichtung (45) ein Zurückziehdraht ist.

27. Stent-Anbringungssystem nach Anspruch 26, bei dem der Katheter eine Führungsdrahtöffnung (45) aufweist, wobei der Führungsdraht inkompressibel und flexibel ist.

28. Stent-Anbringungssystem nach Anspruch 26, wobei der Katheter einen Führungsdraht (45) aufweist, wobei der Führungsdraht an die distale Spitze (25) angebunden ist.

29. Stent-Anbringungssystem nach Anspruch 27, das zusätzlich eine Zurückziehdrahtöffnung aufweist, die teilweise den Zurückziehdraht (45) umschließt und mindestens teilweise durch die proximale äußere Hülle (70) bedeckt ist.

30. Stent-Anbringungssystem nach Anspruch 28, das zusätzlich einen gestärkten Draht aufweist, der entlang dem Katheter positioniert ist.

31. Stent-Anbringungssystem nach Anspruch 27, wobei der Führungsdraht eine polymergekapselte Litze ist.

32. Stent-Anbringungssystem nach Anspruch 27, wobei die Führungsdrahtöffnung (15) eine polymergekapselte Spule ist.

33. Stent-Anbringungssystem nach Anspruch 31, das zusätzlich einen innerhalb der Führungsdrahtöffnung (15) umschlossenen Führungsdraht (45) aufweist.

34. Stent-Anbringungssystem nach Anspruch 33, das eine Vielzahl von Zurückziehdrähten aufweist.

35. Stent-Anbringungssystem nach Anspruch 34, das zusätzlich einen Nackenabschnitt besitzt, der in der zurückziehbaren distalen Hülle (75) angeordnet ist direkt proximal zu der Befestigung zwischen der zurückziehbaren distalen Hülle (75) und dem ringförmigen Bund.

36. Stent-Anbringungssystem nach Anspruch 34, wobei die proximale äußere Hülle (70) ein hoch dichtes Polyethylen aufweist.

37. Stent-Anbringungssystem nach Anspruch 27, wobei das Stent-Anbringungssystem ein über-dem-Draht Katheter ist.

38. Stent-Anbringungssystem nach Anspruch 27, wobei der Katheter 135 cm und die Führungsdrahtöffnung (15) zwischen 5 cm und 35 cm ist.

39. Stent-Anbringungssystem nach Anspruch 21, das zusätzlich einen unter dem Stent innerhalb des Stent-Aufnahmeabschnitts (30) angeordneten Ballon aufweist, wobei nach dem Zurückziehen der zurückziehbaren distalen Hülle (75) der Stent (35) durch Aufblasen des Ballons ausgedehnt wird.

40. Stent-Anbringungssystem nach Anspruch 1, das zusätzlich aufweist:
- eine Führungsdrahtöffnung (15) und eine Zurückziehöffnung (150);
- einen Stent (35), der konzentrisch um den Katheter nahe dem distalen Ende angeordnet ist und einem Verankerungsgerät (140), das fest mit dem Katheter verbunden ist,
- wobei ein Abschnitt der Zurückzieheinrichtung (45) in der Zurückziehöffnung aufgenommen ist und wobei die zusammenlegbare Hülle (50) proximale und distale Enden aufweist und konzentrisch um den Katheter angeordnet ist, wobei das proximale Ende der zusammenlegbaren Hülle (50) an dem Verankerungsgerät (140) befestigt ist und das distale Ende der zusammenlegbaren Hülle (50) an dem proximalen Ende der zurückziehbaren distalen Hülle (40) befestigt ist.

41. Stent-Anbringungssystem nach Anspruch 1, das zusätzlich einen äußeren Schaft (166) aufweist, der auf dem Katheter proximal von der zusammenlegbaren Hülle (174) positioniert ist, wobei die zusammenlegbare Hülle auf dem Katheter proximal zu der zurückziehbaren distalen Hülle positioniert ist.

42. Das Stent-Anbringungssystem nach Anspruch 41, wobei die zurückziehbare distale Hülle eine proximale Hülle (176) und eine Entfaltungshülle (184) aufweist, wobei sowohl die proximale als auch die Entfaltungshülle distal von der zusammenlegbaren Hülle (174) angeordnet sind und die proximale Hülle proximal von der Entfaltungshülle vorgesehen ist, und wobei die proximale Hülle mit der zusammenlegbaren Hülle (174) und der Entfaltungshülle verbunden ist.

43. Stent-Anbringungssystem nach Anspruch 42, das zusätzlich einen distalen äußeren Schaft (68) aufweist, wobei der distale äußere Schaft auf den Katheter zwischen dem proximalen äußeren Schaft und der zusammenlegbaren Hülle (174) angeordnet und mit diesen verbunden ist.

44. Stent-Anbringungssystem nach Anspruch 43, das zusätzlich eine Führungsdrahtöffnung (170) aufweist, die sich innerhalb von dem proximalen Ende des Katheters zu dem distalen Ende des Katheters erstreckt.

45. Stent-Anbringungssystem nach Anspruch 44, das zusätzlich eine distale Spitze (186) aufweist, die an dem distalen Ende der Führungsdrahtöffnung positioniert und an diesem befestigt ist.

46. Stent-Anbringungssystem nach Anspruch 45, wobei die Zurückzieheinrichtung (182) ein Ziehdraht ist und zusätzlich ein Ziehbund (178) vorgesehen ist, der mit der zurückziehbaren distalen Hülle verbunden ist, wobei das distale Ende von dem Ziehdraht mit dem Ziehbund verbunden ist.

47. Stent-Anbringungssystem nach Anspruch 46, das zusätzlich ein Verteilerrohr (164) besitzt, das an dem proximalen Ende des Katheters angeordnet ist und einen Hüllenaktuator (163) aufweist, wobei das proximale Ende des Ziehdrahts (182) mit diesem verbunden ist.

48. Stent-Anbringungssystem nach Anspruch 47, das zusätzlich eine Ziehdrahtöffnung (172) aufweist, die sich innerhalb von distal zu dem Verteilerrohr (164) nach proximal zu dem Ziehbund (178) erstreckt.

49. Stent-Anbringungssystem nach Anspruch 48, wobei der Ziehbund (178) an dem proximalen Ende der Entfaltungshülle befestigt ist.

50. Stent-Anbringungssystem nach Anspruch 49, wobei die Ziehdrahtöffnung (172) unterbrochen ist und an dem proximalen Ende bzw. dem distalen Ende der zusammenlegbaren Hülle (174) fortgesetzt wird.

51. Stent-Anbringungssystem nach Anspruch 50, das zusätzlich einen selbstentfaltenden Stent (35) aufweist, der auf dem distalen Ende der Führungsdrahtöffnung (170) geladen ist, direkt proximal zu dem distalen Ende unter der Entfaltungshülle, wobei der Stent (35) in seinem zusammengelegten Zustand durch die Entfaltungshülle (184) gehalten ist und, wenn der Rückziehdraht (172) proximal zurückgezogen ist, ist die zurückziehbare distale Hülle zurückgezogen, wodurch die zusammenlegbare Hülle (174) zusammengelegt und der Stent (35) zur Anbringung befreit wird.

52. Stent-Anbringungssystem nach Anspruch 51, das zusätzlich einen Puffer (180) aufweist, der an der Führungsdrahtöffnung (170) direkt proportional zu dem geladenen Stent (35) befestigt ist, um eine proximale Bewegung des Stents zu verhindern, wenn die Entfaltungshülle zurückgezogen ist, um den Stent freizugeben.

53. Stent-Anbringungssystem nach Anspruch 52, das zusätzlich Markierungsbänder (194) aufweist, die an der Führungsdrahtöffnung (170) in der Nähe des geladenen Stents (35) befestigt sind derart, daß der Benutzer die genaue Position des Stents innerhalb einer gezielten Vaskularöffnung erkennt.

## Revendications

1. Système de mise en place d'extenseur comprenant :
- un cathéter (5) comprenant une extrémité distale et une extrémité proximale ;
- une partie de réception d'extenseur (30) conçue pour recevoir un extenseur (35) près de l'extrémité distale du cathéter ;
- une gaine distale rétractable (40, 75, 176, 184) présentant une extrémité proximale et une extrémité distale et entourant au moins une partie du cathéter ;
- un moyen de traction (45) ayant une extrémité distale reliée à la gaine distale rétractable (40) en vue d'un retrait de la gaine distale rétractable (40) ;
**caractérisé en ce que** le cathéter comprend
- une gaine pliable (50, 75, 174) disposée concentriquement autour du cathéter, la gaine pliable pouvant se plier sur elle-même dans la direction longitudinale, de manière que lors d'un retrait proximal du moyen de traction (45), la gaine rétractable distale (40, 75, 176, 184) est rétractée, amenant la gaine pliable (50, 75, 174) à se plier axialement sur elle-même.

2. Système de mise en place d'extenseur selon la revendication 1, dans lequel la gaine pliable est positionnée près de la gaine rétractable, le système de mise en place d'extenseur comprenant en outre une gaine extérieure proximale (10) positionnée sur le cathéter près de la gaine pliable (50), la gaine pliable (50) présentant une extrémité distale et une extrémité proximale, l'extrémité distale étant collée à la gaine distale rétractable (40) et l'extrémité proximale étant collée à la gaine extérieure proximale (10).

3. Système de mise en place d'extenseur selon la revendication 2, comprenant en outre un extenseur à auto-expansion (35) chargé sur l'extrémité distale du cathéter, l'extenseur (37) étant maintenu dans son état replié par la gaine distale rétractable (40) et lorsque le moyen de traction (45) est rétracté de façon proximale, la gaine distale (40) est rétractée, amenant la gaine pliable (50) à se plier et à libérer l'extenseur (35) pour une mise en place.

4. Système de mise en place d'extenseur selon la revendication 3, dans lequel le moyen de traction (45) comprend un fil métallique de traction (45).

5. Système de mise en place d'extenseur selon la revendication 4, dans lequel le moyen de traction (45) comprend en outre un collier annulaire (55) entourant le cathéter et longitudinalement mobile le long du cathéter, dans lequel l'extrémité proximale de la gaine distale rétractable (40) est reliée au collier annulaire (55) et l'extrémité distale du fil métallique de traction (45) est reliée au collier annulaire.

6. Système de mise en place d'extenseur selon la revendication 5, comprenant en outre un élément d'arrêt (60) positionné sur le cathéter pour empêcher l'extenseur de se déplacer de manière proximale lorsque la gaine distale est rétractée pour exposer l'extenseur (35).

7. Système de mise en place d'extenseur selon la revendication 6, dans lequel le cathéter comprend une lumière pour fil métallique de guidage (15), la lumière pour fil métallique de guidage étant incompressible et flexible.

8. Système de mise en place d'extenseur selon la revendication 6, dans lequel le cathéter comprend un fil métallique de guidage (20), le fil métallique de guidage étant lié à l'extrémité distale.

9. Système de mise en place d'extenseur selon la revendication 7, comprenant en outre une lumière pour fil métallique de traction qui enferme partiellement le fil métallique de traction (45) et qui est au moins partiellement couverte par la gaine extérieure proximale (10).

10. Système de mise en place d'extenseur selon la revendication 8, comprenant en outre un fil métallique de raidissement positionné longitudinalement le long du cathéter.

11. Système de mise en place d'extenseur selon la revendication 7, dans lequel la lumière pour fil métallique de guidage (15) est une tresse encapsulée dans un polymère.

12. Système de mise en place d'extenseur selon la revendication 7, dans lequel la lumière pour fil métallique de guidage (15) est un enroulement encapsulé dans un polymère.

13. Système de mise en place d'extenseur selon la revendication 11, comprenant en outre un fil métallique de guidage (20) enfermé à l'intérieur de la lumière pour fil métallique de guidage.

14. Système de mise en place d'extenseur selon la revendication 13, comprenant une pluralité de fils métalliques de traction (45) fixés au collier annulaire (55).

15. Système de mise en place d'extenseur selon la revendication 14, dans lequel la gaine distale rétractable (40) comprend en outre une partie de col (62) tout près du raccordement entre la gaine distale rétractable (40) et le collier annulaire (55), qui vient en prise par frottement avec le collier annulaire et aide au retrait de la gaine distale (40).

16. Système de mise en place d'extenseur selon la revendication 14, dans lequel la gaine extérieure proximale (10) comprend un polyéthylène haute densité.

17. Système de mise en place d'extenseur selon la revendication 7, dans lequel le système de mise en place d'extenseur est un cathéter au-dessus du fil métallique.

18. Système de mise en place d'extenseur selon la revendication 7, dans lequel la lumière pour fil métallique de guidage (15) est plus courte que le cathéter.

19. Système de mise en place d'extenseur selon la revendication 18, dans lequel le cathéter mesure 135 cm et la lumière pour fil métallique de guidage (15) mesure entre 5 cm et 35 cm.

20. Système de mise en place d'extenseur selon la revendication 2, comprenant en outre un ballonnet disposé sous l'extenseur, grâce à quoi après que la gaine distale rétractable (40) est rétractée, l'extenseur (35) peut être expansé en gonflant le ballonnet.

21. Système de mise en place d'extenseur selon la revendication 1, comprenant en outre :
- une pointe distale (25) disposée de façon distale par rapport à la partie de réception d'extenseur, la gaine pliable étant
- la gaine distale rétractable (75) et entourant la partie de réception d'extenseur (30).

22. Système de mise en place d'extenseur selon la revendication 21, comprenant en outre une gaine extérieure proximale (70) positionnée sur le cathéter de façon proximale par rapport à la gaine distale rétractable (75), dans lequel la gaine distale rétractable (75) comprend une extrémité proximale, l'extrémité proximale étant collée à la gaine extérieure proximale (70).

23. Système de mise en place d'extenseur selon la revendication 22, comprenant en outre un extenseur à auto-expansion (35) chargé sur l'extrémité distale du cathéter, dans lequel l'extenseur (35) est maintenu à son état replié par la gaine distale rétractable (75) et lorsque le moyen de traction (45) est rétracté de façon proximale, la gaine distale rétractable (75) est rétractée, amenant la gaine distale rétractable à se plier et à libérer l'extenseur (35) pour la mise en place.

24. Système de mise en place d'extenseur selon la revendication 23, comprenant en outre un collier annulaire (55), dans lequel l'extrémité proximale de la gaine distale rétractable (75) et l'extrémité distale du moyen de traction (45) sont reliées au collier.

25. Système de mise en place d'extenseur selon la revendication 24, comprenant en outre un élément d'arrêt (60) positionné pour empêcher l'extenseur de se déplacer de manière proximale lorsque la gaine distale rétractable (75) est rétractée pour exposer l'extenseur (35).

26. Système de mise en place d'extenseur selon la revendication 25, dans lequel le moyen de traction (45) est un fil métallique de traction.

27. Système de mise en place d'extenseur selon la revendication 26, dans lequel le cathéter comprend une lumière pour fil métallique de guidage (15), la lumière pour fil métallique de guidage étant incompressible et flexible.

28. Système de mise en place d'extenseur selon la revendication 26, dans lequel le cathéter comprend un fil métallique de guidage (45), le fil métallique de guidage étant lié à la pointe distale (25).

29. Système de mise en place d'extenseur selon la revendication 27, comprenant en outre une lumière pour fil métallique de traction qui enferme partiellement le fil métallique de traction (45) et qui est au moins partiellement couverte par la gaine extérieure proximale (70).

30. Système de mise en place d'extenseur selon la revendication 28, comprenant en outre un fil métallique de raidissement positionné longitudinalement le long du cathéter.

31. Système de mise en place d'extenseur selon la revendication 27, dans lequel le fil métallique de guidage est une tresse encapsulée dans un polymère.

32. Système de mise en place d'extenseur selon la revendication 27, dans lequel la lumière pour fil métallique de guidage (15) est un enroulement encapsulé dans un polymère.

33. Système de mise en place d'extenseur selon la revendication 31, comprenant en outre un fil métallique de guidage (45) enfermé à l'intérieur de la lumière pour fil métallique de guidage (15).

34. Système de mise en place d'extenseur selon la revendication 33, comprenant une pluralité de fils métalliques de traction.

35. Système de mise en place d'extenseur selon la revendication 34, comprenant en outre une partie de col située dans la gaine distale rétractable (75) tout près de la fixation entre la gaine distale rétractable (75) et le collier annulaire.

36. Système de mise en place d'extenseur selon la revendication 34, dans lequel la gaine extérieure proximale (70) comprend un polyéthylène haute densité.

37. Système de mise en place d'extenseur selon la revendication 27, dans lequel le système de mise en place d'extenseur est un cathéter au-dessus du fil métallique.

38. Système de mise en place d'extenseur selon la revendication 27, dans lequel le cathéter mesure 135 cm et la lumière pour fil métallique de guidage (15) mesure entre 5 cm et 35 cm.

39. Système de mise en place d'extenseur selon la revendication 21, comprenant en outre un ballonnet disposé sous l'extenseur à l'intérieur de la partie de réception d'extenseur (30), grâce à quoi après que la gaine distale rétractable (75) est rétractée l'extenseur (35) est expansé en gonflant le ballonnet.

40. Système de mise en place d'extenseur selon la revendication 1, comprenant en outre :
- une lumière pour fil métallique de guidage (15) et une lumière de traction (150);
- un extenseur (35) disposé concentriquement autour du cathéter près de l'extrémité distale, et un dispositif d'ancrage (140) attaché de manière fixe au cathéter ;
- dans lequel une partie du moyen de traction (45) est logée dans la lumière de traction, et dans lequel la gaine pliable (50) présente des extrémités proximale et distale et est disposée concentriquement autour du cathéter, l'extrémité proximale de la gaine pliable (50) étant fixée au dispositif d'ancrage (140) et l'extrémité distale de la gaine pliage (50) étant fixée à l'extrémité proximale de la gaine distale rétractable (40).

41. Système de mise en place d'extenseur selon la revendication 1, comprenant en outre un manchon extérieur proximal (166) positionné sur le cathéter de manière proximale par rapport à la gaine pliable (174), dans lequel la gaine pliable est positionnée sur le cathéter de manière proximale à la gaine distale rétractable.

42. Système de mise en place d'extenseur selon la revendication 41, dans lequel la gaine distale rétractable comprend une gaine proximale (176) et une gaine de déploiement (184), à la fois la gaine proximale et la gaine de déploiement étant distales par rapport à la gaine pliable (174) et la gaine proximale étant proximale par rapport à la gaine de déploiement, et dans lequel la gaine proximale est reliée à la gaine pliable (174) et à la gaine de déploiement.

43. Système de mise en place d'extenseur selon la revendication 42, comprenant en outre un manchon extérieur distal (168), le manchon extérieur distal étant positionné sur le cathéter entre le manchon extérieur proximal et la gaine pliable (174) et leur étant relié.

44. Système de mise en place d'extenseur selon la revendication 43, comprenant en outre une lumière pour fil métallique de guidage (170), s'étendant à l'intérieur de l'extrémité proximale du cathéter à l'extrémité distale du cathéter.

45. Système de mise en place d'extenseur selon la revendication 44, comprenant en outre une pointe distale (186) positionnée et fixée sur l'extrémité distale de la lumière pour fil métallique de guidage.

46. Système de mise en place d'extenseur selon la revendication 45, dans lequel le moyen de traction (182) est un fil métallique de traction, et comprenant en outre un collier de traction (178) relié à la gaine distale rétractable, l'extrémité distale du fil métallique de traction étant reliée au collier de traction.

47. Système de mise en place d'extenseur selon la revendication 46, comprenant en outre une tubulure (164) positionnée à l'extrémité proximale du cathéter, la tubulure comprenant un actionneur de gaine (163), dans lequel l'extrémité proximale du fil métallique de traction (182) est reliée à celui-ci.

48. Système de mise en place d'extenseur selon la revendication 47, comprenant en outre une lumière pour fil métallique de traction (172) s'étendant à l'intérieur d'un emplacement immédiatement distal par rapport à la tubulure (164) à un emplacement immédiatement proximal par rapport au collier de traction (178).

49. Système de mise en place d'extenseur selon la revendication 48, dans lequel le collier de traction (178) est fixé à l'extrémité proximale de la gaine de déploiement.

50. Système de mise en place d'extenseur selon la revendication 49, dans lequel la lumière pour fil métallique de traction (172) est interrompue et est reprise à l'extrémité proximale et à l'extrémité distale de la gaine pliable (174), respectivement.

51. Système de mise en place d'extenseur selon la revendication 50, comprenant en outre un extenseur à auto-expansion (35) chargé sur l'extrémité distale de la lumière pour fil métallique de guidage (170), de façon immédiatement proximale par rapport à la pointe distale, sous la gaine de déploiement, dans lequel l'extenseur (35) est maintenu dans son état replié par la gaine de déploiement (184), et lorsque le fil métallique de traction (182) est rétracté de manière proximale, la gaine distale rétractable est rétractée, amenant la gaine pliable (174) à se plier et à libérer l'extenseur (35) pour la mise en place.

52. Système de mise en place d'extenseur selon la revendication 51, comprenant en outre un butoir (180) fixé à la lumière pour fil métallique de guidage (170) immédiatement proximale par rapport à l'extenseur chargé (35) pour empêcher l'extenseur de se déplacer de manière proximale lorsque la gaine de déploiement est rétractée pour exposer l'extenseur.

53. Système de mise en place d'extenseur selon la revendication 52, comprenant en outre des bandes repères (194) fixées à la lumière pour fil métallique de guidage (170) à proximité étroite de l'extenseur chargé (35) de façon à permettre à l'utilisateur de positionner de façon précise l'extenseur à l'intérieur d'une lumière vasculaire ciblée.
